(19) Europäisches Patentamt
European Patent Office
Office européen des brevets

(11) Publication number: 0 197 800 B1

(12) # EUROPEAN PATENT SPECIFICATION

(45) Date of publication of patent specification: 13.03.91 (51) Int. Cl.5: C07C 405/00, A61K 31/557

(21) Application number: 86302675.3

(22) Date of filing: 10.04.86

The file contains technical information submitted after the application was filed and not included in this specification

(54) **5-Thiaprostaglandins E1 and process for producing same.**

(30) Priority: 10.04.85 JP 74519/85

(43) Date of publication of application: 15.10.86 Bulletin 86/42

(45) Publication of the grant of the patent: 13.03.91 Bulletin 91/11

(84) Designated Contracting States: CH DE FR GB IT LI NL SE

(56) References cited:
EP-A- 0 112 633
WO-A-86/04330

(73) Proprietor: TEIJIN LIMITED
11 Minamihonmachi 1-chome Higashi-ku
Osaka-shi Osaka 541(JP)

(72) Inventor: Manabe, Kenji
3-3-30, Tamadaira
Hino-shi Tokyo(JP)
Inventor: Okamura, Noriaki
1-37-1, Senkawa-cho
Chofu-shi Tokyo(JP)
Inventor: Kurozumi, Seizi
1-2-28, Higashitokura
Kokubunji-shi Tokyo(JP)

(74) Representative: Arthur, Bryan Edward et al
Withers & Rogers 4 Dyer's Buildings Holborn
London EC1N 2JT(GB)

EP 0 197 800 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

BACKGROUND OF THE INVENTION

1. Field of the Invention

The present invention relates to novel 5-thiaprostaglandins $E_1$ and a process for producing the same. More specifically, the present invention relates to novel 5-thiaprostaglandins $E_1$ useful as medicines for the treatment and prevention of digestive organic diseases such as a duodenal ulcer or a gastric ulcer. The present invention also relates to a process for producing the 5-thiaprostaglandins $E_1$ , in which 5-thia-$\Delta^7$ -prostaglandins $E_1$ having a double bond between the 7- and 8-positions are selectively reduced or 7-hydroxy-5-thiaprostaglandins $E_1$ having a hydroxyl group at the 7-position is dehydroxylated, followed by selectively reducing.

2. Description of the Related Art

Natural prostaglandins are known as local hormones having a biologically and pharmacologically high activity and, therefore, a large number of studies on their derivatives have been made. Among natural prostaglandins, prostaglandin $E_1$ has, for example, a strong blood platelet aggregation inhibition effect and vasodilation effect and, therefore, is used for clinical applications.

The greatest defect of natural prostaglandins, especially prostaglandins $E_1$ (i.e., "PGE$_1$"), is that they can not be orally administered, because they are rapidly metabolized when orally administered and, therefore, must be usually administered by an intravenous or intraarterial injection.

Various studies have been made of the conventional manner of preparing synthetic prostaglandins by replacing one or two carbon atoms forming the skeleton of natural prostaglandins with a sulfur atom(s).

For example, there are known IS-thiaprostaglandin $E_2$ or $F_{2\alpha}$ wherein the carbon atom at the I-position of natural prostaglandins is replaced with a sulfur atom (i.e., since the sulfur atom is present at the I-position, "IS" is nominated the head of the name of the compound and similarly the number of the position at which sulfur is substituted and the suffix S are used at the head of the compound) (J. Org. Chem., 40 , 521, (I975) Japanese Unexamined Patent Publication (Kokai) No. 53-34747), 3S-II-deoxyprostaglandin $E_1$ (Tetrahadron Letters, I975 , 765; and J. Med. Chem., 20 , 1663 (I977)), 7S-prostaglandins $F_{1\alpha}$ (J. Amer. Chem. Soc., 96 , 6757 (I974)), 9S-prostaglandins $E_1$ (Tetahedron Letters, I974 , 4267 and 4459; Tetrahedron Letters, I976 , 4793; and Heterocycles, 6, I097 (I977)), IIS-prostaglandins $E_1$ or $F_{1\alpha}$ (Tetrahedron Letters, I975 , II65), I3S-prostaglandins- E or F (U.S. Patent No. 4,080,458), I5S-prostaglandins $E_2$ (Tetrahedron Letters, I977 , I629), 4S, 6S, or 7S-prostaglandins $E_1$ U.S. Patent No. 4,466,980), and 5S-prostaglandins $E_1$ (European Patent Publication No. A3I,426). Furthermore, EP-A 0II2633 discloses certain 5-S prostaglandins having a (protected) I6-hydroxy group, useful as anti-ulcer agents.

Among the objects of the present invention are the provision of novel 5-thiaprostaglandins $E_1$ and a process for the preparation thereof, especially for oral administration for the treatment or prevention of digestive organic diseases such as duodenal ulcer or gastric ulcer.

Other objects and advantages of the present invention will be apparent from the following description.

In accordance with the present invention, there are provided.5-thiaprostaglandins $E_1$ having the general formula (I):

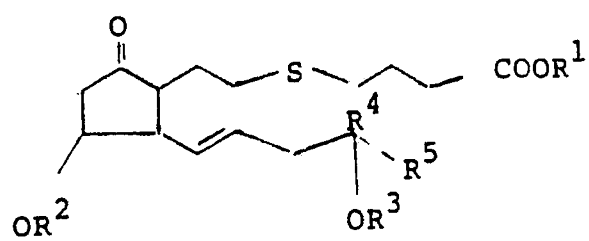

... (I)

wherein $R^1$ represents a hydrogen atom, a $C_1$-$C_{10}$ alkyl group, a substituted or unsubstituted phenyl group, a substituted or unsubstituted $C_3$-$C_{10}$ cycloalkyl group, a substituted or unsubstituted phenyl ($C_1$-$C_2$) alkyl group, or one equivalent cation; $R^2$ and $R^3$, which may be the same or different, represent a hydrogen atom, a tri($C_1$-$C_7$)hydrocarbon silyl group, or a group forming an acetal linkage together with an oxygen

atom of a hydroxyl group; $R^4$ represents a hydrogen atom, a methyl group, an ethyl group or a vinyl group; and $R^5$ represents a linear or branched $C_3$-$C_8$ alkyl group, a linear or branched $C_3$-$C_8$ alkenyl group, a linear or branched $C_3$-$C_8$ alkynyl group, a phenyl group which may be substituted, a phenoxy group which may be substituted, a $C_3$-$C_{10}$ cycloalkyl group which may be substituted, or a linear or branched $C_1$-$C_5$ alkyl group substituted with a $C_1$-$C_6$ alkoxy group, a phenyl group which may be substituted, a phenoxy group which may be substitued, or a $C_3$-$C_{10}$ cycloalkyl group which may be substituted.

## DESCRIPTION OF THE PREFERRED EMBODIMENT

In the formula (I), $R^1$ represents a hydrogen atom, a $C_1$-$C_{10}$ alkyl group, a substituted or unsubstituted phenyl group, a substituted or unsubstituted $C_3$-$C_{10}$ cycloalkyl group, a substituted or unsubstituted phenyl ($C_1$-$C_2$) alkyl group, one equivalent cation.

The $C_1$-$C_{10}$ alkyl groups may be linear on branched and may include, for example, methyl, ethyl, n-propyl, iso-propyl, n-butyl, sec-butyl, tert-buryl, n-pentyl, n-hexyl, n-heptyl, n-octyl, n-nonyl, and n-decyl.

The substituent of the subsbituted phenyl is preferably, for example, a halogen atom, a hydroxy group, a $C_2$-$C_7$ acyloxy group, a $C_1$-$C_4$ alkyl group which may be substituted with a halogen atom, a $C_1$-$C_4$ alkoxy group which may be substituted with a halogen atom, a nitrile group, a carboxyl group or a ($C_1$-$C_6$) alkoxycarbonyl group. The halogen atom is, for example, fluorine, chlorine or bromine, and particularly preferably, fluorine or chlorine. The $C_2$-$C_7$ acyloxy group may include, for example, acetoxy, propionyloxy, n-butyryloxy, iso-butyryloxy, n-valeryloxy, iso-valeryloxy, caproyloxy, enanthyloxy or benzoyloxy.

The $C_1$-$C_4$ alkyl groups which may be substituted with a halogen may preferably include, for example, methyl, ethyl, n-propyl, iso-propyl, n-butyl, chloromethyl, dichloromethyl, and trifluoromethyl. The $C_1$-$C_4$ alkoxy groups which may be substituted with a halogen may preferably include, for example, methoxy, ethoxy, n-propoxy, iso-propoxy, n-butoxy, chloromethoxy, dichloromethoxy and trifluoromethoxy. The ($C_1$-$C_6$) alkoxycarbonyl groups may include, for example, methoxycarbonyl, ethoxycarbonyl, butoxycarbonyl and hexyloxycarbonyl.

The substituted phenyl group may have l to 3, preferably one, substituents as mentioned above.

The substituted or unsubstituted $C_3$-$C_{10}$ cycloalkyl group may be substituted with a similar substituent as mentioned above or may be a unsubstituted $C_3$-$C_{10}$ , preferably $C_5$-$C_6$ , cycloalkyl group, such as cyclopropyl, cyclopentyl, cyclohexenyl, cycloheptyl, cyclooctyl, and cyclodecyl.

The substituted or unsubstituted phenyl ($C_1$-$C_2$) alkyl groups may be substituted with a similar substitutent as mentioned above or may include, for example, unsubstituted benzyl, $\alpha$-phenethyl and $\beta$-phenethyl.

The one equivalent cations may include, for example, ammonium cations such as $NH_4$ , tetramethylammonium, monomethylammonium, dimethylammonium, trimethylammonium, benzylammonium, phenethylammonium, morpholinium cation, monoethanolammonium and piperidinium cation; alkali metal cations such as Na and K ; and divalent or trivalent metal cations such as l/2 $Ca^2$ , l/2 $Mg^{2+}$· l/2 $Zn^2$ and l/3 $Al^{3+}$.

$R^1$ is preferably a hydrogen atom, a $C_1$-$C_{10}$ alkyl group, or one equivalent cation.

In the formula (I), $R^2$ and $R^3$ may be the same or different and represent a hydrogen atom, a tri($C_1$-$C_7$) hydrocarbon silyl group or a group forming an acetal linkage together with an oxygen atom of a hydroxyl group.

The tri($C_1$-$C_7$) hydrocarbon silyl groups may preferably include, for example, a tri($C_1$-$C_4$) alkylsilyl such as trimethylsilyl, triethylsilyl, and t-butyldimethylsilyl; a diphenyl ($C_1$-$C_4$) alkylsilyl such as diphenylmethylsilyl and t-butyldiphenylsilyl; di($C_1$-$C_4$) alkylphenylsilyl such as dimethylphenylsilyl; or tribenzylsilyl. Of these tri($C_1$-$C_7$) hydrocarbon silyl groups, tri($C_1$-$C_4$) alkylsilyl groups, diphenyl ($C_1$-$C_4$) alkylsilyl group, and di($C_1$-$C_4$) alkylphenylsilyl groups, especially trimethylsilyl and t-butyldimethylsilyl are preferable.

The group forming an acetal linkage together with an oxygen atom of the hydroxyl group may include, for example, methoxyethyl, l-ethoxyethyl, 2-methoxy-2-propyl, 2-ethoxy-2-propyl, (2-methoxyethoxy) methyl, benzyloxymethyl, 2-tertrahydropyranyl, 2-tetrahydrofuranyl, or 6,6-dimethyl-3-oxa-2-oxobicylo [3.l.0] hexa-4-yl. Of these, 2-tetrahydropyranyl, 2-tetrahydrofuranyl l-ethoxyethyl, 2-ethoxy-2-propyl, (2-methoxyethoxy) methyl or 6,6-dimethyl-3-oxa-2-oxobicyclo [3.l.0] hexa-4-yl is especially preferable.

In the formula (I), $R^4$ represents a hydrogen atom, a methyl group or a vinyl group. Especially, a methyl group is preferable.

In the formula (I) $R^5$ represents a linear or branched $C_3$-$C_8$ alkyl group a linear or branched $C_3$-$C_8$ alkenyl group, a linear or branched alkynyl group, a phenyl group which may be substituted, a phenoxy group which may be substituted, a $C_3$-$C_{10}$ cycloalkyl group which may be substituted, or a linear or

branched $C_1$-$C_5$ alkyl group which may be substituted with a $C_1$-$C_6$ alkoxy group, a phenyl group which may be substituted, a phenoxy group which may be substituted or a $C_3$-$C_{10}$ cycloalkyl group whcih may be substituted.

The linear or branched $C_3$-$C_8$ alkyl groups may include n-propyl, n-butyl, n-pentyl, n-hexyl, l-methyl-l-butyl, and 2-methyl-l-butyl, preferably n-butyl, n-pentyl, and l-methyl-l-butyl, particularly preferably, butyl.

The linear or branched $C_3$-$C_8$ alkenyl groups may include, for example, l-butenyl, 2-butenyl and l-pentenyl. The linear or branched $C_3$-$C_8$ alkynyl groups may include l-butynyl, 2-butynyl and l-pentynyl.

The substituent of a phenyl group which may be substituted, a phenoxy group which may be substituted and a $C_3$-$C_{10}$ cycloalkyl group which may be substituted may be a similar substituent as mentioned for the substituent of a substituted phenyl group of $R^1$ . The unsubstituted $C_3$-$C_7$ cycloalkyl group may include preferably a $C_4$-$C_7$ , more preferably a $C_5$-$C_6$ , cycloalkyl group, for example, cyclopropyl, cyclopentyl, cyclohexyl, cycloheptyl, cyclooctyl and cyclodecyl.

The $C_1$-$C_6$ alkoxy groups in the linear or branched $C_1$-$C_5$ alkyl group which may be substituted with a $C_1$-$C_6$ alkoxy group, a phenyl group which may be substituted, a phenoxy group which may be substituted on a $C_3$-$C_{10}$ cycloalkyl group which may be substituted may include, for example, methoxy, ethoxy, propoxy, isopropoxy, butoxy, t-butoxy, and hexyloxy. The phenyl group which may be substituted and the phenoxy group which may be substituted may preferably include those mentioned above. The $C_3$-$C_{10}$ cycloalkyl groups which may be substituted may preferably include those mentioned above. The linear or branched $C_1$-$C_5$ alkyl groups ` substituted with these groups may include, for example, methyl, ethyl, n-propyl, isopropyl, n-butyl, iso-butyl, sec-butyl, t-butyl and pentyl. The substituents may be attached to the alkyl group at any position.

As $R^5$, butyl, pentyl, l-methyl-l-butyl, 2-methyl-l-butyl, cyclopentyl, cyclohexyl and phenyl are preferable.

The 5-thiaprostaglandins $E_1$ according to the present invention may include those having the following general formula (I') and those having the following general formula (I')ent:

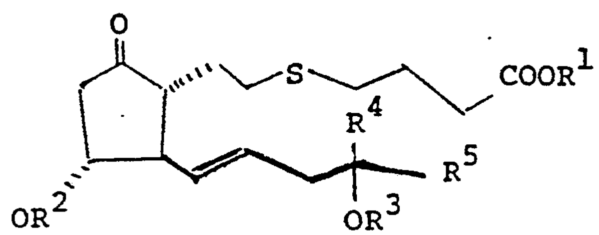

... (I')

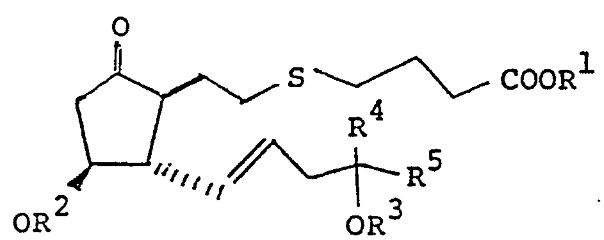

... (I')ent

wherein $R^1$ , $R^2$ , $R^3$ , $R^4$ , and $R^5$ are the same as defined above. That is, in the general formula (I), the steric configuration of the substtuents attached to the cyclopentanone ring is the same as those of natural prostaglandin $E_1$, whereas the general formula (I)ent represents an enantiomer thereof. The present invention includes any of the above-mentioned optical isomers or mixtures thereof in any ratio.

Preferable examples of the novel 5-thiaprostaglandins $E_1$ of the formula (I) according to the present invention are as follows:

(1) 15-deoxy-16-hydroxy-16-methyl-5-

thiaprostaglandin $E_1$

(2) 15-deoxy-16-hydroxy-5-thiaprostaglandin $E_1$

(3) 15-deoxy-16-hydroxy-20-methyl-5-thiaprostaglandin $E_1$

(4) 15-deoxy-16-hydroxy-16-vinyl-5-thiaprostaglandin $E_1$

(5) 15-deoxy-16-hydroxy-16-ethyl-5-thiaprostaglandin $E_1$

(6) The enantiomers of the compounds (1) to (5)

(7) The methyl esters of the compounds (1) to (5)

(8) The sodium salt of the compounds (1) to (5)

(9) The ethers of the compounds (1) to (8) in which the hydroxyl groups at the ll-and l6-positions are protected with a t-butyldimethyl silyl group and/or a 2-tetrahydropyanyl group, and/or a trimethylsilyl group. It should be, of course, noted that the scope of the present invention is by no means limited to these compounds. For example, the optical isomers, at the l6-position, of the compounds (l) to (9) and the enantiomers of all these compounds are also included within the scope of the present invention.

The 5-prostaglandins $E_1$ according to the present invention having the general formula (I) or their enantiomers or mixtures thereof in any ratio can be prepared as follows.

That is, 7-hydroxy-5-thiaprostaglandins $E_1$ having the general formula (III):

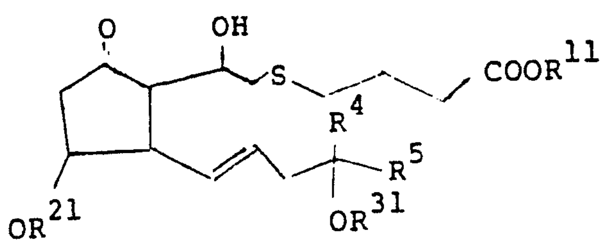

... (III)

wherein $R^4$ and $R^5$ are the same as defined above, $R^{11}$ represent a hydrogen atom, a $C_1$-$C_{10}$ alkyl group, a substituted or unsubstituted phenyl group, a substituted or unsubstituted $C_3$-$C_{10}$ cycloalkyl group, or a substituted or unsubstituted phenyl ($C_1$-$C_2$) alkyl group, and $R^{21}$ and $R^{31}$ which may be the same or different and are a tri ($C_1$-$C_7$) hydrocarbon silyl group or a group forming an acetal linkage together with an oxygen atom of a hydroxyl group are reacted with a reactive derivative of an organic sulfonic acid in the presence of a basic compound to form the corresponding 7-organosulfonyloxy-5-thiaprostaglandins $E_1$, and the resultant 7-organosulfonyloxy-5-thiaprostaglandins $E_1$ are treated with a basic compound, followed by optionally subjecting the resultant product to deprotection (i.e., removal of a protective group) and/or hydrolysis and/or salt-formation, thereby forming 5-thia-$\Delta^7$-prostaglandins having the general formula (II)

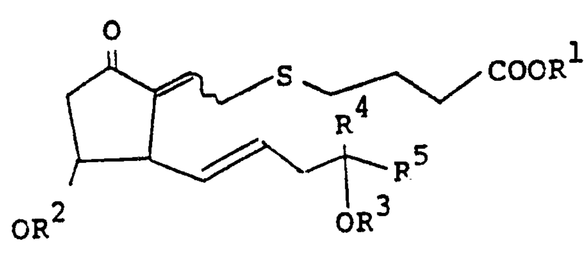

... (II)

wherein $R^1$, $R^2$, $R^3$, $R^4$, and $R^5$ are as defined above and the indication

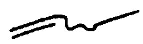

represents a Z- form, a E- form, or a mixture of a Z- form and a E- form in any ratio, and, then, the resultant 5-thia-$\Delta^7$-prostaglandins are selectively reduced, followed by optionally subjecting the resultant product to deprotection and/or hydrolysis and/or salt-formation. Thus, the desired 5-thiaprostaglandins $E_1$ having the general formula (I) can be prepared.

The 7-hydroxy-5-thiaprostaglandins $E_1$ of the formula (III) can be readily obtained, for example, by a one stage reaction from the protected 4-hydroxy-2-cyclopentenone, at a high yield as follows.

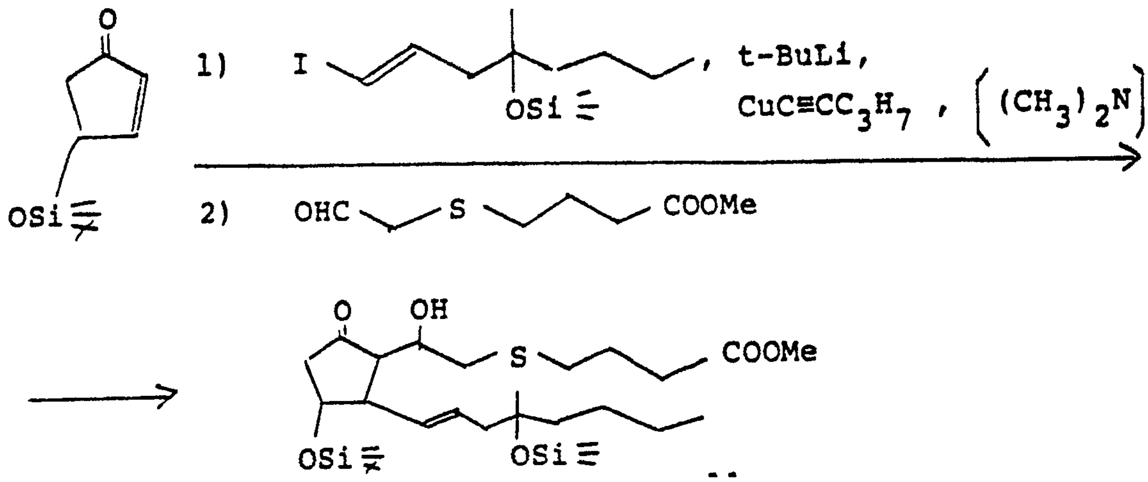


In the general formula (III), $R^{11}$ represents a hydrogen atom or an alkyl group having I to 10 carbon atoms similar to those mentioned above. preferable $R^{11}$ is a methyl group.

In the general formula (III), $R^{21}$ and $R^{31}$ may be the same or different and represent a tri ($C_1$-$C_7$) hydrocarbon silyl group or a group forming an acetal linkage together with an oxygen atom of a hydroxyl group as mentioned above. Typical examples of such groups are also similar to those mentioned above.

In the first reaction, the 7-hydroxy-5-thiaprostaglandins $E_1$ of the formula (III) are allowed to be reacted with the reactive derivatives of organosulfonic acids in the presence of basic compounds such as organic amines, preferably tertiary organic amines.

Examples of such organic amines are trimethyl amine, triethyl amine, iso-propyldimethyl amine, di-isopropylcyclohexyl amine, di-isopropylethyl amine, pyridine, 2,6-lutidine, 2,4,6-collidine, 4-(N,N-dimethyl) aminopyridine, l,5-diazabicyclo [4,3,0] nona-5-ene, and l,4-diazabicyclo [2,2,2 ]octane. Of these amines, the use of triethyl amine, pyridine, 2,6-lutidine, and 4-(N,N-dimethyl) aminopyridine is preferable.

The reaction is preferably carried out in an inert non-protonic organic medium. Examples of such solvents are halogenates hydrocarbons such as dichloromethane, chloroforn, carbon tetrachloride, and l,2-dichloroethane; ethers such as ethyl ether, tetrahydrofuran, and dioxane; aromatic hydrocarbons such as benzene, toluene, xylene; aliphatic hydrocarbons such as hexane, pentane, and cyclohexane; esters such as ethyl acetate; dimethyl formamide; and hexamethylphosphor amide. Of these soluents, the use of halogenated hydrocarbons is especially preferable.

The reactive derivatives of the organosulfonic acids preferably include organosulfonic acid halides and organosulfonic acid anhydrides.

Typical examples of the organosulfonic halides are methanesulfonic chloride, ethanesulfonic bromide, n-butanesulfonic chloride, t-buctanesulfonic chloride, trifluoromethanesulfonic chloride, nonafluorobutanesulfonic chloride, t-butanesulfonic chloride, trifluoromethanesulfonic chloride, nonafluorobutanesulfonic chloride, benzenesulfonic chloride, p-toluenesulfonic chloride, 4-bromobenzenesulfonic chloride, pentafluorobenzenesulfonic chloride, 4-chloro-3-nitrobenzenesulfonic chloride, 2,3,4-trichlorobenzenesulfonic chloride, and 2-phenylethanesulfonic chloride. Of these compounds, the use of methanesulfonic chloride, trifluoromethanesulfonic chloride, and nonafluorobutanesulfonic anhydride, benzenesulfonic anhydride, p-toluenesulfonic chloride is preferable.

Typical examples of the organosulfonic anhydrides are methanesulfonic anhydride, ethanesulfonic anhydride, trifluoromethanesulfonic anhydride, p-toluenesulfonic anhydride, the acid anhydride of mixed acids of trifluoromethanesulfonic acid and benzenesulfonic acid, the acid anhydride of mixed acids of trifluoromethanesulfonic acid and p-toluenesulfonic acid, and the acid anhydride of mixed acids of trifluoromethesulfonic acid and 4-nitrobenzenesulfonic acid. Of these acid anhydrides, the use of

methanesulfonic anhydride, trifluromethanesulfonic anhydride, and p-toluenesulfonic anhydride is preferable.

The above-mentioned reaction of the hydroxyl group at the 7-position of the 7-hydroxy-5-thiaprostaglandins $E_1$ and the reactive derivatives of organosulfonic acids stoichiometrically occurs in an equimolar reaction. Practically, l to 50 times, preferably l to l0 times by mol, based on one mole of the 7-hydroxy-5-thiaprostaglandins $E_1$ , of the reactive derivatives of the organosulfonic acids are used. The reaction is generally carried out at a temperature of -20 °C to l00 °C, preferably 0 °C to 50 °C for l to 60 hours, preferably 2 to l2 hours. Thus, in the first reaction, the corresponding 7-organosulfonyloxy-5-thiaprostaglandins $E_1$ (i.e., the compound in which the hydroxyl group at the 7-position of the 7-hydroxy-5-thiaprostaglandins (III) is substituted with an organosulfonyloxy group) is formed. The 7-organosulfonyloxy-5-thiaprostaglandins $E_1$ thus formed are treated with basic compounds (i.e., the second reaction). As a result, the 7-organosulfonyloxy-5-thiaprostaglandins $E_1$ release the organosulfonic group to form a double bond between the carbon atoms of the 7- and 8- positions. Thus, the 5-thia-$\Delta^7$-prostaglandins $E_1$ of the formula (II) are obtained. The second reaction can be carried out by using similar basic compounds at a temperature similar to that of the above-mentioned first reaction.

The first and second reactions may be carried out either by isolating the 7-organosulfonyloxy-5-thiaprostaglandins $E_1$ formed in the first reaction or by using the same reaction system without separating the resultant 7-organosulfonyloxy-5-thiaprostaglandins $E_1$ from the reaction mixture. In the latter case, the second reaction may be carried out with or without adding the fresh basic compounds after the first reaction.

The basic compounds used in the first and second reactions may be the same or different. For example, the first reaction is carried out by using a relatively weak base such as pyridine, triethyl amine, or isopropyldimethyl amine and the second reaction is carried out by using a relatively strong base such as 4-dimethylamino pyridine and l,5-diazabicyclo[4,3,0]nona-3-ene.

After the reaction, the reaction mixture can be treated in any conventional manner (e.g., extraction, washing, drying, concentration, and chromatography, alone or in any combination) to separate and purify the desired compound.

The desired compounds obtained above can be subjected to the deprotection reaction and/or salt-formation reaction.

The removal of the protective group ($R^{21}$ and $R^{31}$) of a hydroxyl group (i.e., the deprotection reaction), when the protective group is a group forming an acetal linkage together with an oxygen atom of a hydroxyl group, is conveniently carried out in a reaction solvent such as water, tetrahydrofuran, ethyl ether, dioxane, acetone, and acetonitrile, in the presence of a catalyst such as acetic acid, the pyridinium p-toluensulfonate or a cation exchange resin. The deprotection reaction is usually carried out at a temperature ranging from -78 °C to +30 °C for approximately l0 minutes to 3 days.

In the case where the protective group is a tri ($c_1$-$C_7$) hydrocarbon silyl group, the reaction is carried out by using, for example, acetic acid, tetrabutyl ammonium fluoride, or cesium fluoride, preferably tetrabutyl ammonium fluoride, or cesium fluoride, preferably in the presence of a basic compound such as triethylamine. Thus, the protective group is removed the solvents usable in the practice of the deprotection reaction in the case of the protective group being tri ($c_1$-$C_7$) hydrocarbon-silyl group include, for example, water, tetrahydrofuran, ethyl ether, dioxane, acetone, and acetonitrile, preferably tetrahydrofuran, ethyl ether, dioxane, acetone, and acetonitrile. The reaction temperature and the reaction time are similar to those of the above-mentioned deprotection reaction in the case of the acetal linkage.

The hydrolysis of the 5-thia-$\Delta^7$-prostaglandin $E_1$ (i.e., the hydrolysis of the ester group in the case of $R^{11}$ = a $C_1$-$C_{10}$ alkyl group, a substituted or unsubstituted phenyl group, a substituted or unsubstituted $C_3$-$C_{10}$ cycloalkyl group, or a substituted or unsubstituted phenyl ($C_1$-$C_2$) alkyl group) can be carried out, for example, by treating the compound in water or an aqueous solvent with an enzyme such as lipase at a temperature of -l0 °C to +60 °C for approximately l0 minutes to 24 hours.

The salt-forming reaction of the 5-thia-$\Delta^7$-prostaglandins $E_1$ (i.e., the salt-forming reaction of the carboxyl group in the case of $R^{11}$ in the l-position being a hydrogen atom) is known per se, and is carried out by a neutralization reaction with an inorganic basic compound, such as potassium hydroxide, sodium hydroxide, and sodium carbonate, or an organic basic compound, such as ammonia, trimethylamine, monoethanolamine, and morpholine, in an amount substantially equal to that of the 5-thia-$\Delta^7$-prostaglandin $E_1$ according to a conventional method.

The desired 5-thia-$\Delta^7$-prostaglandins $E_1$ obtained above are then selectively reduced. The selective reduction is carried out, preferably, by a reduction method using zinc type reducing agents such as zinc-silver reducing agents and zinc-copper reducing agents, or a reduction method using an organotin hydrogenide compound. When the zinc reducing agent is used, the reaction is carried out also in the presence of glacial acetic acid. Although there are no specific limitations to the amount of the zinc reducing

agents used, the reducing agent is generally used in an amount of l to l000 moles, preferably 5 to 200 moles, based on l mole of the starting compound (II) in the presence of l to 2000 moles, preferably l0 to l000 moles, also based on l mole of the starting compound (II). The solvents usable in the reduction reaction preferably include, for example, alcohols such as methanol, ethanol, isopropyl alcohol; dimethoxyethane; dimethylformamide; dimethylsulfoxide; acetic acid; or the mixtures thereof. The especially preferable solvents are methanol, isopropyl alcohol, and acetic acid. There are no specific limitations in the amount of the solvents used, as long as the solvent is present in an amount sufficient to allow the desired reaction to proceed rapidly or smoothly. Generally, the solvent is used in an amount of l to 2000 times by volume, preferably 5 to l000 times by volume, based on the starting compound. The reaction is generally carried out at a temperature of 0 °C to ll5 °C, preferably l0 °C to 80 °C, for l to 48 hours, preferably 3 to 24 hours, although these conditions may vary depending upon, for example, the kinds of the starting compounds, reagents, and solvents and also the reaction conditions. The progress of the reaction is followed or monitored by, for example, a thin-layer chromatography. After the completion of the reaction, the desired 5-thiaprostaglandins $E_1$ having the general formula (I) can be recovered and purified by treating the reaction mixture in a conventional manner. For example, the reaction mixture is treated by extraction, washing, drying, condensing, or chromatography, or any combination thereof.

The organotin compounds (i.e., organotin hydrides) usable in the above-mentioned reduction include, for example, tributyltin hydride, triphenyltin hydride, and triethyltin hydride. These organotin hydrides are generally used in an amount of l to l000 equivalent amount, preferably l to l00 equivalent amount, based on the starting compound (II). The reaction solvents such as alcohols may be used, but, generally speaking, the use of no solvent reaction is preferable. The reaction can be smoothly progressed by using a free radical type initiator or photo irradiation. Typical examples of such initiators are $\alpha,\alpha'$-azobisisobutyronitrile and di-t-butyl peroxide. These initiators are generally used in an amount of 0.0l to l0 equivalent, preferably 0.l to l equivalent, based on the starting material (II). The reaction temperature is generally 0 °C to 200 °C, preferably 30 °C to l30 °C. The progress of the reaction can be followed or monitored by, for example, a thin layer chromatography method. When the reaction is ionically proceeded, a palladium catalyst such as tetrakis (triphenylphospine) palladium is used in an amount of, generally 0.00l to 0.l equivalent, preferably 0.005 to 0.02 equivalent.

The reaction is carried out generally at a temperature of 0 °C to 200 °C, preferably l0 °C to 50 °C. After the reaction, the desired 5-thiaprostaglandins $E_1$ having the general formula (I) are recovered and purified by treating the reaction mixture in any conventional manner, for example, by filtration, concentration, chromatography, or the like, or any combination thereof.

The desired compounds thus obtained may be subjected to the above-mentioned deprotection and/or hydrolysis and/or salt formation reaction, if necessary. Thus, the desired 5-thiaprostaglandins $E_1$ of the formula (I) can be obtained at a high yield under moderate reaction conditions according to the present invention.

Of the 5-thiaprostaglandins $E_1$ according to the present invention, l6-hydroxy-l6-methyl-5-thiaprostaglandin $E_1$ methyl ester exhibits a strong anti-ulcer activity and prevents the ulcer generation at a preventive rate of about 50% for an oral administration of 40 $\mu$g/kg in the case of rat indomethacin ulcer. Furthermore, in the case of rat ethanol ulcer, the ulcer generation can be prevented at a prevention rate of about 50% for an oral administration of 4 $\mu$g/kg.

EXAMPLES

The present invention will now be further illustrated in detail by, but is by no means limited to, the following Examples.

Reference Example l

Synthesis of ll-t-butyldimethylsilyloxy-l5-deoxy-7-hydroxy-l6-methyl-l6-trimethylsilyloxy-5-thiaprostaglandin $E_1$ methyl ester

A 3.74 g (ll mmol) amount of 4-trimethylsilyloxy-4-methyl-l-iodo-l-octene was dissolved in l7 ml of anhydrous ether. The resultant solution was added to a l.7 M solution of l2.9 ml (22 mmol) of t-butyl lithium in 7.5 ml of anhydrous ether at a temperature of -72 °C under an argon atmosphere and the mixture was then stirred for 2 hours.

Then, a solution of l.44 g (ll mmol) of l-pentynyl copper (I) and 4.00 ml (22 mmol) of hexamethylphosphorus triamide in l3 ml of anhydrous ether was added and the mixture was further stirred at a temperature of -72 °C for one hour.

To the resultant reaction mixture, a solution of 2.l2 g (l0 mmol) of 4-t-butyldimethylsilyloxy-2-cyclopen-

tenone in l8 ml of anhydrous ether was added and the mixture was stirred at a temperature of -72°C for l5 minutes and -50°C for 50 minutes. Then, a solution of l.94 g (ll mmol) of methyl 7-oxo-5-thiaheptanate in l2.5 ml of anhydrous ether at a temperature of -50°C was added and the mixture was stirred at a temperature of -72°C for l0 minutes and -30°C to -40°C for 45 minutes.

The reaction mixture was poured into a acetic acid-sodium aoetate buffer solution having an adjusted pH of 4 and the mixture was extracted with hexane. The extract was dried over anhydrous magnesium sulfate and, after concentrating, the extract was subjected to silica gel chromatography. Thus, the desired ll-t-butyldimethylsilyloxy-l5-deoxy-7-hydroxy-l6-methyl-l6-trimethylsilyloxy-5-thiaprostaglandin $E_1$ methyl was separated and purified. The yield was 56%.

The analytical data of the desired compound are as follows.

TLC Rf; 0.45 (solvent; hexane: ethyl acetate = 3:1)

NMR (δ ppm in $CDCl_3$);

0.9 (s, 9H), 0.9-1.5 (m, 12H),
1.7-3.1 (m, 14H), 3.7 (s, 3H),
3.7-4.4 (m, 3H), 5.5 (m, 2H).

Reference Example 2

Synthesis of (l6 S )-ll-t-butyldimethylsilyloxy-l5-deoxy-7-hydroxy-l6-methyl-l6-trimethylsilyloxy-5-thiaprostaglandin $E_1$ methyl ester

A 2.04 g (6 mmol) amount of (4 S )-4-trimethylsilyloxy-4-methyl-l-iodo-l-octene was dissolved in l0 ml of anhydrous ether. The resultant solution was charged with a 2.0 M solution of 6 ml (l2 mmol) of t-butyl lithium at a temperature of -72°C under an argon atmosphere and the mixture was then stirred for one hour.

Then, a solution of 867 mg (6 mmol) of l-pentynyl copper (l) and l.96 g (l2 mmol) of hexamethylphosphrus triamide in l2 ml of anhydrous ether was added and the mixture was further stirred at a temperature of -72°C for one hour.

To the resultant reaction mixture, a solution of l.l6 g (5.45 mmol) of (4 R )-t-butyldimethylsilyloxy-2-cyclopentenone in l8 ml of anhydrous ether was added and the mixture was stirred at a temperature of -72°C for l5 minutes and -50°C for 50 minutes. Then, a solution of l.06 g (6 mmol) of methyl 7-oxo-5-thiaheptanate in l2.5 ml of anhydrous ether at a temperature of -50°C was added and the mixture was stirred at a temperature of -72°C for l0 minutes and -50°C to -40°C for 45 minutes.

The reaction mixture was poured into a acetic acid-sodium acetate buffer solution having an adjusted pH of 4 and the mixture was extracted with hexane. The extract was dried over anhydrous magnesium sulfate and, after concentrating, the extract was subjected to silica gel chromatography. Thus, the desired (l6 S )-ll-t-butyldimethylsilyloxy-l5-deoxy-7-hydroxy-l6-methyl-l6-trimethylsilyloxy-5-thiaprostaglandin $E_1$ methylester was separated and purified. The yield was 2.23 g (68%).

The analytical data of the desired compound are as follows.

TLC Rf; 0.45 (solvent; hexane: ethyl acetate = 3:1)

NMR (δ ppm in $CDCl_3$);

0.9 (s, 9H), 0.9-1.5 (m, 12H),
1.7-3.1 (m, 14H), 3.7 (s, 3H),
3.7-4.4 (m, 3H), 5.5 (m, 2H).

Example l

Synthesis of ll-t-butyldimethylsilyloxy-l5-deoxy-l6-methyl-l6-trimethylsilyloxy-5-thia-$\Delta^7$-prostaglandin $E_1$ methyl ester

A 2.42 g (4.02 mmol) amount of ll-t-butyldimethylsilyloxy-l5-deoxy-7-hydroxy-l6-methyl-l6-trimethylosilyloxy-5-thiaprostaglandin $E_1$ methyl ester was dissolved in l5 ml of anhydrous dichloromethane. The resultant solution was charged with l.32 g (l0.85 mmol) of 4-dimethylamine pyridine at a temperature of 0-l0 °C and, then, with 0.40 ml (5.23 mmol) of methanesulfonyl chloride at a temperature of 0 °C. The mixture was stirred at room temperature for 2.5 hours. Water was added to the reaction mixture and the mixture was dried over anhydrous sodium sulfate. After concentrating, the mixture was subjected to silica gel chromatography to separate and purify the resultant compound.

Thus, l5.3 g (64% yield) of the desired ll-t-butyldimethylsilyloxy-l5-deoxy-l6-methyl-l6-trimethylsilyloxy-5-thia-$\Delta^7$-prostaglandin $E_1$ methyl ester was obtained. The analytical data are as follows.

TLC Rf: 0.65 (solvent; hexane: ethyl acetate = 3:1)

NMR ($\delta$ ppm in $CDCl_3$);

0.9 (s, 9H), 0.9-1.5 (m, 12H),
1.7-2.8 (m, 10H), 3.0-3.2 (m, 2H),
3.4 (m, 1H), 3.6 (s, 3H), 4.2 (m, 1H),
5.4 (m, 2H), 6.5-6.7 (m, 1H).

Example 2

Synthesis of ll-t-butyldimethylsilyloxy-l5-deoxy-l6-methyl-l6-trimethylsilyloxy-5-thiaprostaglandin $E_1$ methyl ester

A 500 mg (0.86 mmol) amount of ll-t-butyldimethyl-silyloxy-l5-deoxy-l6-methyl-l6-trimethylsilyloxy-5-thia-$\Delta^7$-prostaglandin $E_1$ methyl ester was charged with 2.76 ml of tributyltin hydride and 3 drops of di-t-butyl peroxide and the mixture was stirred at a temperature of l00 °C for 2.5 hours. The mixture was cooled to room temperature and was subjected to silica gel chromatography to separate and purify the desired compound. Thus, l52 mg (30% yield) of ll-t-butyldimethylsilyloxy-l5-deoxy-l6-methyl-l6-trimethylsilyloxy-5-thiaprostaglandin $E_1$ methyl ester was obtained.

The analytical data are as follows:

TCL Rf: 0.35 (Solvent; hexane: diethylether = 2:1)

Example 3

Synthesis of l5-deoxy-l6-hydroxy-l6-methyl-5-thiaprostaglandin $E_1$ methyl ester

A l50 mg (0.26 mmol) amount of ll-t-butyldimethyl-silyloxy-l5-deoxy-l6-methyl-l6-trimethylsilyloxy-5-thiaprostaglandin $E_1$ methyl ester was dissolved in 2.3 ml of acetonitrile. Then, ll6 ml of pyridine was further added and the mixture was cooled to a temperature of 0 °C. Then, 235 $\mu$l of hydrogen fluoride-pyridine was added and the mixture was stirred at room temperature for l.5 hours. Thereafter, 4.2 ml of a saturated sodium bicarbonate solution and 0.94 g of sodium bicarbonate were added and the mixture was extracted with ethyl acetate.

The reaction mixture was subjected to silica gel chromatography to separate and purify the desired compound. Thus, 90 mg (yield = 88%) of the desired l5-deoxy-l6-hydroxy-l6-methyl-5-thiaprostaglandin $E_1$ methyl ester was obtained.

The analytical data of the compound are as follows.

TLC Rf: 0.35 (Solvent; hexane: ethyl acetate = 1:4)

NMR (δ ppm in $CDCl_3$ , 400 MHz);
0.9 (t, 3H), 1.18 (s, 3H),
1.3-1.5 (6H), 1.88 (m, 2H),
2.3-2.4 (m, 4H), 2.7-2.8 (w, 2H),
3.67 (s, 3H), 4.07 (t, 1H),
5.4-5.8 (m, 2H), 2.5-2.7 (w, 1H)

IR ($cm^{-1}$, neat);
3450, 2950, 2875, 1740, 1440
1370-800

Mass (70 eV, m/e);
400 ($M^+$-17), 383, 282, 231

Example 4

Synthesis of (16 S )-11-t-butyldimethylsilyloxy-15-deoxy-16-methyl-16-trimethylsilyloxy-5-thia-$\Delta^7$-prostaglandin $E_1$ methyl ester

A 1.8 9 (2.99 mmol) amount of (16 S )-11-t-butyl-dimethylsilyloxy-15-deoxy-7-hydroxy-16-methyl-16-trimethylsilyloxy-5-thiaprostaglandin $E_1$ methyl ester was dissolved in 15 ml of anhydrous dichloromethane. The resultant solution was charged with 1.8 g (2.99 mmol) of 4-dimethylamine pyridine at a temperature of 0 °C and, then, with 0.30 ml (3.88 mmol) of methanesulfonyl chloride at a temperature of 0 °C. The mixture was stirred at room temperature for 4.5 hours. Water was added to the reaction mixture and the mixture was extracted with hexane. The extract was dried over anhydrous sodium sulfate. After concentrating, the mixture was subjected to silica gel chromatography to separate and purify the resultant compound.

Thus, 1.43 g (82% yield) of the desired (16 S )-11-t-butyldimethylsilyloxy-15-deoxy-16-methyl-16-trimethylsilyloxy-5-thia-$\Delta^7$-prostaglandin $E_1$ methyl ester was obtained. The analytical data are as follows.

TLC Rf: 0.65 (solvent; hexane: ethyl acetate = 3.1)

NMR (δ ppm in $CDCl_3$);
0.9 (s, 9H), 0.5-1.5 (m, 12H),
1.7-2.8 (m, 10H), 2.0-3.2 (m, 2H),
3.4 (m, 1H), 3.6 (s, 3H), 4.2 (m, 1H),
5.4 (m, 2H), 6.5-6.7 (m, 1H).

Example 5

Synthesis of (16 S )-11-t-butyldimethylsilyloxy-15-deoxy-16-methyl-16-trimethylsilyloxy-5-thiaprostaglandin $E_1$ methyl ester

A 2.1 g (3.6 mmol) amount of (16 S )-11-t-butylmethyl-silyloxy-15-deoxy-16-methyl-16-trimethylsilyloxy-5-thia-$\Delta^7$-prostaglandin $E_1$ methyl ester was charged with 126 mg (0.108 mmol) of tetrakis triphenylphosphine palladium and, after the system was replaced with nitrogen, 9 ml of tributyltin hydride was gradually

dropwise added thereto. The mixture was stirred at a temperature of 10-30°C for 10 minutes. The mixture was then charged with water and was extracted with hexane. The extract was subjected to silica gel chromatography to separate and purify the desired compound. Thus, 1.23 g (59% yield) of 11-t-butyldimethylsilyloxy-15-deoxy-16-methyl-16-trimethylsilyloxy-5-thiaprostaglandin $E_1$ methyl ester was obtained.

The analytical data are as follows:

TCL Rf: 0.35 (Solvent; hexane: diethylether = 2:1)

NMR (δ ppm in $CDCl_3$);

0.9 (s, 9H), 0.9-1.5 (m, 9H), 1.15 (s, 3H), 1.6-2.7 (m, 16H), 3.6 (s, 3H),

3.7-4.1 (m, 1H), 5.2-5.4 (m, 2H)

Example 6

Sunthesis of (16 S)-15-deoxy-16-hydroxy-16-methyl-5-thiaprostaglandin $E_1$ methyl ester

A 1.17 g (1.99 mmol) amount of (16 S)-11-t-butyl-dimethylsilyloxy-15-deoxy-16-methyl-16-trimethylsilyloxy-5-thiaprostaglandin $E_1$ methyl ester was dissolved in 24 ml of acetonitrile. Then, 1.2 ml of pyridine was further added and the mixture was cooled to a temperature of 0°C. Then, 2.4 ml of hydrogen fluoride-pyridine was added and the mixture was stirred at room temperature for 3 hours. Thereafter, the mixture was neutralized with sodium bicarbonate solution and was extracted with ethyl acetate.

The reaction mixture was subjected to silica gel chromatography to separate and purify the desired compound. Thus, 596 mg (yield = 75%) of the desired (16 S)-15-deoxy-16-hydroxy-16-methyl-5-thiaprostaglandin $E_1$ methyl ester was obtained.

The analytical data of the compound are as follows.

TLC Rf: 0.35 (Solvent; hexane: ethyl acetate = 1:4)

NMR(δ ppm in $CDCl_3$, 400 MHz);

0.9 (t, 3H), 1.18 (s, 3H),
1.3-15.(6H), 1.88 (m, 2H),
2.3-2.4 (m, 4H), 2.7-2.8 (w, 2H),
3.67 (s, 3H), 4.07 (t, 1H),
5.4-5.8 (m, 2H), 2.5-2.7 (w, 1H)

IR($cm^{-1}$, neat);

3450, 2950, 2875, 1740, 1440
1370-800

Example 7

(i) Measurement of inhibitory activity on ethanol ulcer

An ethanol ulcer test was carried out by the following procedure.

SD-strain rats (body weight 200 to 220 g; 7 week old) were fasted for 24 hours, and a solution of the test compound in physiological saline buffered with phosphate buffer at pH 7.5 was orally administered. Thirty minutes after the administration, 75% ethanol was orally administered in a dose of l ml/kg. One hour later, the abdomen was incised to isolate the stomach. The corpus was observed with a stereomicroscope, and the lengths of ulcers were measured. The total of these lengths was defined as the ulcer index.

From this measurement, the inhibition of ulcer formation by the test compound was calculated, to determine the $IC_{50}$ value. The result is shown in Table I.

(ii) Measurement of inhibitory activity on indomethacin ulcer

The inhibiting effect on ulcer formation induced by indomethacin was examined by using Wister male rats (7 weeks old, body weight 220 g) which were fasted for 24 hours, except for water, and then subjected to experiment.

The sample compound to be tested was dissolved in a phosphoric acid buffer (pH 7.4) containing 0.9% NaCl and the solution was orally administered to the rats. 30 minutes after the administration, indomethacin was orally administered to the rats at a dose of 20 mg/kg. 5 hours after the administratin of the indomethacin, the rats were killed and the ulcer formation in the stomach was examined by determining the length of the ulcer formation portion under a stereomicroscope. From this measurement, the inhibition of ulcer formation by the sampel compound was calculated to determine the $IC_{50}$ values. The result is shown in Table I.

(iii) Measurement of activity on blood presure

The actions of the 5-thiaprostaglandin of the invention on the blood pressure was examined by intravenous injection under anesthesia.

Urehtane (500 mg/kg) and α-chloralose (l00 mg/kg) were intraperitoneally administered to male wister rats weighing about 250 g. The rats were anesthetized and fixed in place.

The test compound was dissolved in a small amount of ethanol and diluted with physiological saline to adjust the final ethanol concentration to not more than 5%. The solution was intravenously injected into the rats through a catheter inserted into the femoral vein.

The blood pressure of the rats was measured by a pressure transducer through a catheter inserted into the carotid artery of the rats.

The action of the test compound on the blood pressure was expressed as the dosage ($ED_{20}$ , μg/kg) of the test compound which caused a 20% lowering of the mean blood pressure before administration of the compound.

The results are shown in Table I.

(iv) Measurement of in vitro inhibitory activity of platelet aggregation

The in vitro platelet aggregation inhibiting activity of the compound of the invention was examined by using rabbits. Blood was withdrawn from the ear vein of Japanese domestic white male rabbits weighing 2.5 to 3.5 kg. A mixture of a 3.8% trisodium citrate solution and the blood in a ratio of l:9 was centrifuged at a speed of l000 rpm for l0 minutes. The upper layer was separated as platelet-rich plasma (PRP). The lower layer was further centrifuged at a speed of 2800 rpm for l0 minutes. The upper layer was separated as platelet-poor plasma (PPP). The number of plate lets was adjusted to 6 x $l0^5/\mu l$ to 7 x $l0^5/\mu l$ by diluting the PRP with PPP. 25 microliters of the test compound prepared as shown below was added with an amount of 25 to 250 microliters of PRP after the adjustment, and the mixture was pre-incubated at 37°C for 2 minutes, and then l0 μM (final) of ADP was added. Changes in transmission were recorded by using an aggregometer.

The drug was dissolved in ethanol to a concentration of l0 mg/ml, and seccussively diluted with phosphate buffer (pH 7.4) prior to use.

The rate of inhibition of platelet aggregation was determined from the following equation.

$$\text{Inhibition rate (\%)} = \left(1 - \frac{T}{T_0}\right) \times 100$$

$T_0$: the transmittance of the system containing the phosphate buffer,

T : the transmittance of the system to which the test drug was added.

The minimum concentration of the drug which inhibited more than 50% of platelet aggregation was shown as an $IC_{50}$ value.

The result is shown in Table I.

(v) Measurement of extra vivo inhibitory activity of platelet aggregation

The extra vivo platelet aggregation inhibitory activity of the 5-thiaprostaglandin $E_1$ derivatives of the invention were measured by using guinea pigs. A test drug prepared as a 10% ethanol solution of the compound of the invention in ethanol diluted with l0% ethanol-0.5% Tween*80 in saline were orally administered in a dose of 0.3-3 mg/kg, and as a control l0% ethanol-0.5% Tween*80 in saline was orally administered in a dose of l ml/kg to Hartley-strain guinea pigs having a body weight of 350 to 450 g which had been fasted for l7 hours. l hour after the administration of the compound, blood was drawn off by carbiac puncture using trisodium citrate so that the ratio of the blood to 3.8% trisodium citrate was 9:l. The blood was centrifuged at a speed of 700 rpm for l0 minutes. The upper layer was separated as platelet-rich plasma (PRP). The lower layer was further centrifuged at a speed of 2800 rpm for l0 minutes. The upper layer was separated as platelet poor plasma (ppp]. A 250 microliter amount of the resulting PRP was taken into an aggregometer cuvette and * Registered Trade Mark incubated at 37° C for 2 minutes. Then, 25 microliters of a l0M ADP disodium solution adjusted by dissolving with 0.lM tris-HCl buffer (pH 8.0) and 25 microliters of l00 g/ml collagen solution adjusted by dissolving with physiological saline, was added. The aggregation curve was recorded for 5 minutes. The maximum degree of aggregation of platelets within this time period was read, and the rate of inhibition of platelet aggregation was calculated from the following equation.

$$\text{Inhibition of platelet} = \left(1 - \frac{T_D}{T_C}\right) \times 100$$

$T_C$: the transmittance of a control group (to which only the 30% ethanol solution was administered),

$T_D$: the transmittance of a drug administered group.

The results are shown in Table I.

Table 1

| | Ethanol Ulcer $IC_{50}$ (µg/kg, P.O.) | Indoethacin Ulcer $IC_{50}$ (µg/kg, P.O.) | Pressure $ED_{20}$ (µg/kg, P.O.) | Blood Inhibition of Platelet in vitro $IC_{50}$ (µg/kg, i.v.) | Aggregation extra vivo (µg/ml) | collagen | ADP |
|---|---|---|---|---|---|---|---|
| Compound of example 6 | 4 | · | 40 | 2.1 | 60 | N.E. | N.E. |

N.E.: no-effect

As shown in Table 1, the compound of the present invention has stronger antiulcer activity in comparison with an inhibiting platelet aggregation activity, and therefore has a high anti-ulcer activity selectively.

## Claims

**1.** 5-Thiaprostaglandins $E_1$ having the general formula (I):

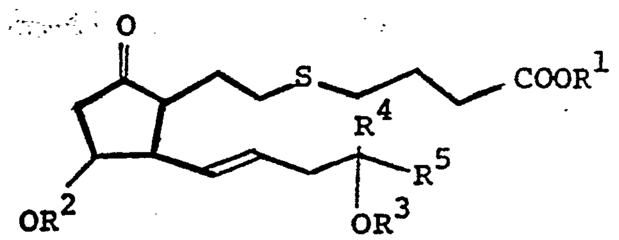

... (I)

wherein $R^1$ represents a hydrogen atom, a $C_1$-$C_{10}$ alkyl group; a substituted or unsubstituted phenyl group, a substituted or unsubstituted $C_3$-$C_{10}$ cycloalkyl group, a substituted or unsubstituted phenyl ($C_1$-$C_2$) alkyl group, or one equivalent cation; $R^2$ and $R^3$, which may be the same or different, represent a hydrogen atom, a tri($C_1$-$C_7$)hydrocarbon silyl group, or a group forming an acetal linkage together with an oxygen atom of a hydroxyl group; $R^4$ represents a hydrogen atom, a methyl group, an ethyl group or a vinyl group; and $R^5$ represents a linear or branched $C_3$-$C_8$ alkyl group, a linear or branched $C_3$-$C_8$ alkenyl group, a linear or branched $C_3$-$C_8$ alkynyl group, a phenyl group which may be substituted, a phenoxy group which may be substituted, a $C_3$-$C_{10}$ cycloalkyl group which may be substituted, or a linear or branched $C_1$-$C_5$ alkyl group substituted with a $C_1$-$C_6$ alkoxy group, a phenyl group which may be substituted, a phenoxy group which may be substituted, or a $C_3$-$C_{10}$ cycloalkyl group which may be substituted.

2. The 5-thiaprostaglandins $E_1$ according to claim I, wherein $R^1$ is a hydrogen atom, a $C_1$-$C_{10}$ alkyl, or one equivalent cation.

3. The 5-thiaprostaglandins $E_1$ according to claim I, wherein $R^2$ and $R^3$ may be the same or different, and are hydrogen atom, a tri($C_1$-$C_4$)alkylsilyl group, a diphenyl($C_1$-$C_4$)alkylsilyl group, a 2-tetrahydropyranyl group, a 2-tetrahydrofuranyl group, a I-ethoxyethyl group, a 2-ethoxy-2-propyl group, a (2-methoxyethoxy) methyl group, or a 6,6-dimethyl-3-oxa-2-oxobicyclo [0,I,0]hexa-4-yl group.

4. The 5-thiaprostaglandins $E_1$ according to claim I, wherein $R^5$ is a butyl group, a pentyl group, a I-methyl-I-butyl group, a 2-methyl-I-butyl group, a cyclopentyl group, a cyclohexyl group, or a phenyl group.

5. The 5-thiaprostaglandins $E_1$ according to claim I, where $R^4$ is methyl group.

6. A process for producing 5-thiaprostaglandins $E_1$ having the general formula (I):

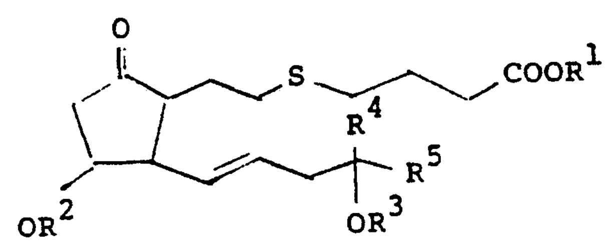

... (I)

wherein $R^1$ represents a hydrogen atom, a $C_1$-$C_{10}$ alkyl group, a substituted or unsubstituted phenyl group, a substituted or unsubstituted $C_3$-$C_{10}$ cycloalkyl group, a subsbituted or unsubsituted phenyl ($C_1$-$C_2$) alkyl group, or one equivalent cation; $R^2$ and $R^3$, which may be the same or different, represent a hydrogen atom, a tri($C_1$-$C_7$)hydrocarbon silyl group, or a group forming an acetal linkage together with an oxygen atom of a hydroxy1 group; $R^4$ represents a hydrogen atom, a methyl group, an ethyl group or a vinyl group; and $R^5$ represents a linear or branched $C_3$-$C_8$ alkyl group, a linear or branched $C_3$-$C_8$ alkenyl group, a linear or branched $C_3$-$C_8$ alkynyl group, a phenyl group which may be substituted, a phenoxy group which may be substituted, a $C_3$-$C_{10}$ cycloalkyl group which may be substituted, or a linear or branched $C_1$-$C_5$ alkyl group substituted with a $C_1$-$C_6$ alkoxy group, a phenyl group which may be substituted, a phenoxy group which may be substituted, or a $C_3$-$C_{10}$ cycloalkyl group which may be substituted, comprising selectively reducing 5-thia-$\Delta^7$-prostaglandins $E_1$ having the general formula (II):

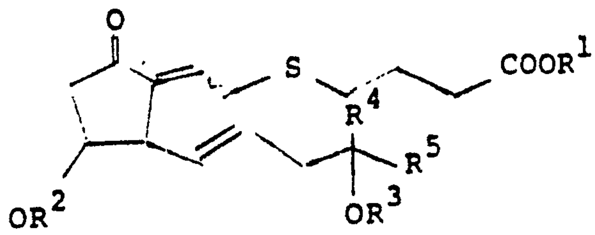

... (II)

wherein $R^1$, $R^2$, $R^3$, $R^4$, or $R^5$ are the same as defined above and the indication

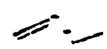

represents a Z- form, a E-form, or a mixture of a Z- form and a E- form in any ratio, and optionally subjecting the resultant product to deprotection and/or hydrolysis and/or salt-formation.

**7.** The process according to claim 6, wherein the selective reduction is carried out in the presence of a zinc type reducing agent or an organotin hydride compound.

**8.** The process according to claim 6, wherein $R^1$ is a $C_1$-$C_{10}$ alkyl group.

**9.** The process according to claim 6, wherein $R^2$ and $R^3$ may be the same or different, and are hydrogen atom, a tri($C_1$-$C_4$)alkylsilyl group, a diphenyl($C_1$-$C_4$)-alkylsilyl group, a 2-tetrahydropyranyl group, a 2-tetrahydrofuranyl group, a l-ethoxyethyl group, a 2-ethoxy-2-propyl group, a (2-methoxyethoxy) methyl group, or a 6,6-dimethyl-3-oxa-2-oxobicyclo[3,l,0]hexa-4-yl group.

**10.** A process for producing 5-thiaprostaglandins $E_1$ having the general formula (I):

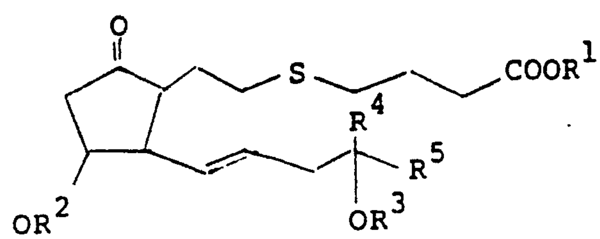

... (I)

wherein $R^1$ represents a hydrogen atom, a $C_1$-$C_{10}$ alkyl group, a substituted or unsubstituted phenyl group, a substituted or unsubstituted $C_3$-$C_{10}$ cycloalkyl group, a substituted or unsubstituted phenyl ($C_1$-$C_2$) alkyl group, or one equivalent cation; $R^2$ and $R^3$, which may be the same or different, represent a hydrogen atom, a tri($C_1$-$C_7$)hydrocarbon silyl group, or a group forming an acetal linkage together with an oxygen atom of a hydroxyl group; $R^4$ represents a hydrogen atom, a methyl group, an ethyl group or a vinyl group; and $R^5$ represents a linear or branched $C_3$-$C_8$ alkyl group, a linear or branched $C_3$-$C_8$ alkenyl group, a linear or branched $C_3$-$C_8$ alkynyl group, a phenyl group which may be substituted, a phenoxy group which may be substituted, a $C_3$-$C_{10}$ cycloalkyl group which may be substituted, or a linear or branched $C_1$-$C_5$ alkyl group substituted with a $C_1$-$C_6$ alkoxy group, a phenyl group which may be substituted, a phenoxy group which may be substituted, or a $C_3$-$C_{10}$ cycloalkyl group which may be substitued, comprising the steps of:

reacting a 7-hydroxy-5-thiaprostaglandins $E_1$ having the general formula (III):

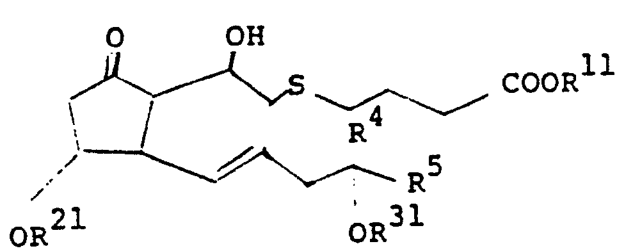

... (III)

wherein $R^4$ and $R^5$ are the same as defined above, $R^{11}$ represent a hydrogen atom, a $C_1$-$C_{10}$ alkyl group, a substituted or unsubstituted phenyl group, a substituted or unsubstituted $C_3$-$C_{10}$ cycloalkyl group, or a substituted or unsubstituted phenyl ($C_1$-$C_2$) alkyl group, and $R^{21}$ and $R^{31}$ which may be the same or different and are a tri ($C_1$-$C_7$) hydrocarbon silyl group or a group forming an acetal linkage together with an oxygen atom of a hydroxyl group with a reaction derivative of an organic sulfonic acid in the presence of a basic compound to form the corresponding 7-organosulfonyloxy-5-thiaprostaglandins $E_1$ ,

treating the resultant 7-organosulfonyloxy-5-thiaprostaglandins $E_1$ with a basic compound, followed by optionally subjecting the resultant product to deprotection and/or hydrolysis and/or salt formation thereby forming 5-thia-$\Delta^7$-prostaglandins having the general formula (II)

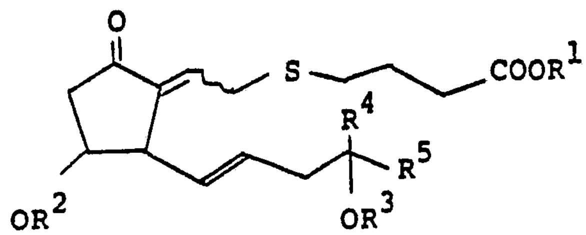

... (II)

wherein $R^1$, $R^2$, $R^3$, $R^4$, and $R^5$ are as defined above and the indication

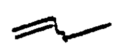

represents a Z- form, a E- form, or a mixture of a Z- form and a E- form in any ratio,

selectively reducing the resultant compound (II), and

optionally subjecting the resultant reduced product to deprotection and/or hydrolysis and/or salt-formation.

**11.** The process according to claim I0, wherein the reactive derivative of the organic sulfonic acid is methanesulfonyl chloride, p-toluenesulfonyl chloride, or methanesulfonic acid anhydride.

**12.** The process according to claim I0, wherein the basic compound is an amine.

## Revendications

**1.** 5-thiaprostaglandines $E_1$ représentées par la formule générale (I)

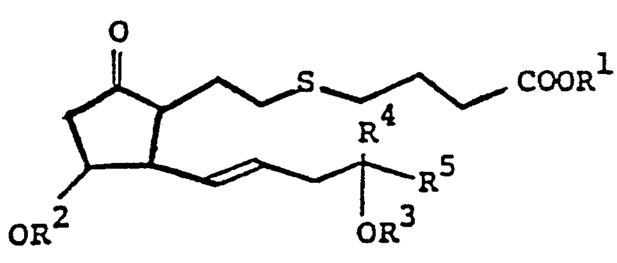

... (I)

dans laquelle R' représente un atome d'hydrogène, un groupement alkyle en $C_1$-$C_{10}$, un groupement phényle subtitué ou non substitué, un groupement cycloalkyle en $c_3$-$c_{10}$ substitué ou non substitué un groupement phényl-($C_1$-$C_2$)-alkyle substitué ou non substitué ou un cation équivalent un; $R^2$ et $R^3$, qui peuvent être identiques ou différents, représentent chacun un atome d'hydrogène, un groupement silyle tri-($C_1$-$C_7$)-hydrocarboné ou un groupement formant une liaison acétal avec un atome d'oxygène d'un groupement hydroxy; $R^4$ représente un atome d'hydrogène, un groupement méthyle, un groupement éthyle ou un groupement vinyle; et $R^6$ représente un groupement alkyle en $C_3$-$C_8$ linéaire ou ramifié, un groupement alcényle en $C_3$-$C_8$ linéaire ou ramifié, un groupement alcynyle en $C_3$-$C_8$ linéaire ou ramifié, un groupement phényle qui peut être substitué, un groupement phénoxy qui peut être

substitué, un groupement cycloalkyle en $C_3$-$C_{10}$ qui peut être substitué ou un groupement alkyle en $C_1$-$C_5$ linéaire ou ramifié substitué par un groupement alcoxy en $C_1$-$C_6$ par un groupement phényle qui peut être substitué, par un groupement phénoxy qui peut être substitué ou par un groupement cyloalkyle en $C_3$-$C_{10}$ qui peut être substitué.

2. 5-thiaprostaglandine $E_1$ selon la revendication I, dans lesquelles $R^1$ est un atome d'hydrogène, un groupement alkyle en $C_1$-$C_{10}$ ou un cation équivalent un.

3. 5-thiaprostaglandines $E_1$ selon la revendication I, dans lesquelles $R^2$ et $R^3$ peuvent être identiques ou différents et représentent chacun un atome d'hydrogène, un groupement tri-($C_1$-$C_4$)-alkylsilyle, un groupement diphényl-($C_1$-$C_4$)-alkylsilyle, un groupement 2-tétrahydropyrannyle, un groupement 2-tétrahydrofurannyle, un groupement l-éthoxyéthyle un groupement 2-éthoxy-2-propyle, un groupement (2-methoxyéthoxy)-méthyle ou un groupement 6,6-diméthyl-3-oxa-2-oxobicyclo[3,l,0]hexa-4-yle.

4. 5-thiaprostaglandines $E_1$ selon la revendication I, dans lesquelles $R^5$ est un groupement butyle un groupement pentyle, un groupement l-méthyl-l-butyle, un groupement 2-méthyl-l-butyle un groupement cyclopentyle, un groupement cyclohexyle ou un groupement phényle.

5. 5-thiaprostaglandines $E_1$ selon la revendication I, dans lesquelles $R^4$ est un groupement méthyle.

6. Procédé de préparation de 5-thiaprostaglandines $E_1$ représentées par la formule générale (I)

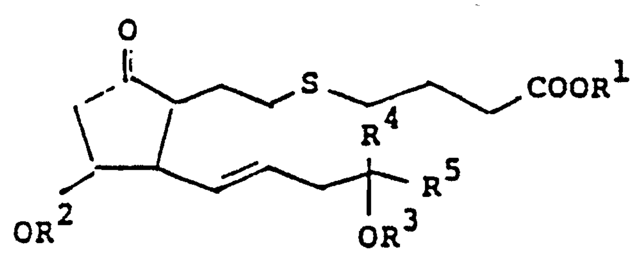

... (I)

dans laquelle $R^1$ représente un atome d'hydrogène un groupement alkyle en $C_1$-$C_{10}$, un groupement phényle substitué ou non substitué un groupement cycloalkyle en $C_3$-$C_{10}$ substitué ou non substitué un groupement phényl-($C_1$-$C_2$)-alkyle substitué ou non substitué ou un cation équivalent un; $R^2$ et $R^3$, qui peuvent être identiques ou différents, représentent chacun un atome d'hydrogène un groupement silyle tri-($C_1$-$C_7$)-hydrocarboné ou un groupement formant une liaison acétal avec un atome d'oxygène d'un groupement hydroxy; $R^4$ représente un atome d'hydrogène, un groupement méthyle un groupement éthyle ou un groupement vinyle; et $R^5$ représente un groupement alkyle en $C_3$-$C_8$ linéaire ou ramifié un groupement alcényle en $C_3$-$C_8$ linéaire ou ramifié un groupement alcynyle en $C_3$-$C_8$ linéaire ou ramifié un groupement phényle qui peut être substitué un groupement phénoxy qui peut être substitué un groupement cycloalkyle en $C_3$-$C_{10}$ qui peut être substitué ou un groupement alkyle en $C_1$-$C_6$ linéaire ou ramifié substitué par un groupement alcoxy en $C_1$-$C_6$, par un groupement phényle qui peut être substitué, par un groupement phénoxy qui peut être substitué ou par un groupement cycloalkyle en $C_3$-$C_{10}$ qui peut être substitué. comprenant les opérations consistant à réduire sélectivement des 5-thia-$\Delta^7$-prostaglandines $E_1$ de formule générale (II)

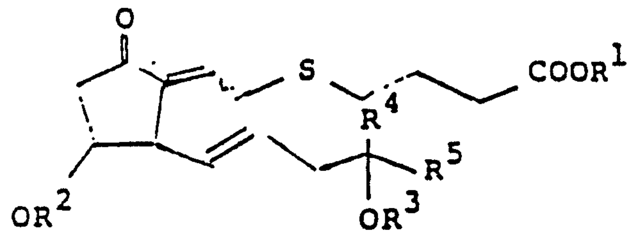

... (II)

dans laquelle $R^1$, $R^2$, $R^3$, $R^4$ et $R^5$ sont tels que définis précédemment et l'indication

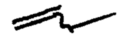

représente une forme Z, une forme E ou un mélange d'une forme Z et d'une forme E dans n'importe quel rapport et à soumettre éventuellement le produit résultant à une déprotection et/ou à une hydrolyse et/ou à une salification.

7. Procédé selon la revendication 6, dans lequel la réduction sélective est effectuée en présence d'un agent réducteur du type zinc ou d'un hydrure organostannique.

8. Procédé selon la revendication 6, dans lequel R' est un groupement alkyle en $C_1$-$C_{10}$.

9. Procédé selon la revendication 6, dans lequel $R^2$ et $R^3$ peuvent être identiques ou différents et représentent chacun un atome d'hydrogène, un groupement tri-($C_1$-$C_4$)-alkylsilyle, un groupement diphényl-($C_1$-$C_4$)-alkylsilyle, un groupement 2-tétrahydropyrannyle, un groupement 2-tétrahydrofurannyle, un groupement l-éthoxyéthyle, un groupement 2-éthoxy-2-propyle, un groupement (2-méthoxyéthoxy)-méthyle ou un groupement 6,6-diméthyl-3-oxa-2-oxobicyclo[3,l,0]hexa-4-yle.

10. Procédé de préparation de 5-thiaprostaglandines $E_1$ représentées par la formule générale (I)

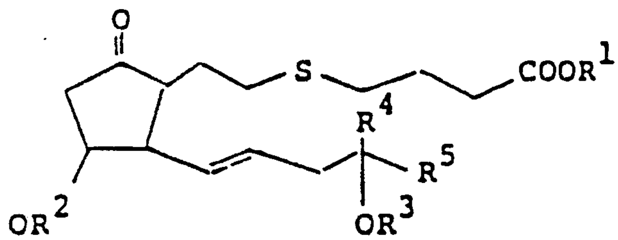

... (I)

dans laquelle $R^1$ représente un atome d'hydrogène, un groupement alkyle en $C_1$-$C_{10}$, un groupement phényle substitué ou non substitué, un groupement cycloalkyle en $C_3$-$C_{10}$ substitué ou non substitué, un groupement phényl-($C_1$-$C_2$)-alkyle substitué ou non substitué ou un cation équivalent un; $R^2$ et $R^3$, qui peuvent être identiques ou différents, représentent chacun un atome d'hydrogène, un groupement silyle tri-($C_1$-$C_7$)-hydrocarboné ou un groupement formant une liaison acétal avec un atome d'oxygène d'un groupement hydroxy; $R^4$ représente un atome d'hydrogène, un groupement méthyle, un groupement éthyle ou un groupement vinyle; et $R^5$ représente un groupement alkyle en $C_3$-$C_8$ linéaire ou ramifié, un groupement alcényle en $C_3$-$C_8$ linéaire ou ramifié, un groupement alcynyle en $C_3$-$C_8$ linéaire ou ramifié, un groupement phényle qui peut être substitué, un groupement phénoxy qui peut être substitué, un groupement cycloalkyle en $C_3$- $C_{10}$ qui peut être substitué ou un groupement alkyle en $C_1$-$C_5$ linéaire ou ramifié substitué par un groupement alcoxy en $C_1$-$C_6$, par un groupement phényle qui peut être substitué, par un groupement phénoxy qui peut être substitué ou par un groupement cycloalkyle en $C_3$-$C_{10}$ qui peut être substitué. comprenant les opérations consistant à mettre en réaction une 7-hydroxy-5-thiaprostaglandines $E_1$ représentée par la formule générale (III)

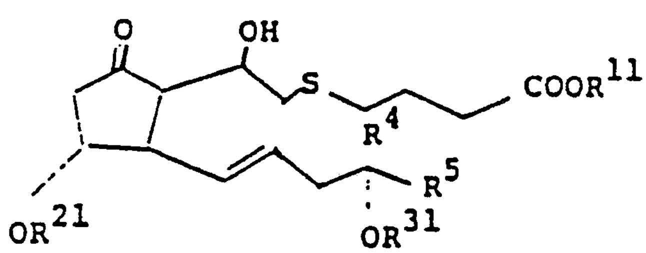

... (III)

dans laquelle $R^4$ et $R^5$ sont tels que définis précédemment, $R^{11}$ représente un atome d'hydrogène, un groupement alkyle en $C_1$-$C_{10}$, un groupement phényle substitué ou non substitué, un groupement cycloalkyle en $C_3$-$C_{10}$ substitué ou non substitué ou un groupement phényl-($C_1$-$C_2$)-alkyle, et $R^{21}$ et $R^{31}$, qui peuvent être identiques ou différents, représentent chacun un groupement silyle tri-($C_1$-$C_7$)-hydrocarboné ou un groupement formant une liaison acétal avec un atome d'oxygène d'un groupement

hydroxy, avec un dérivé réactif d'un acide organique sulfonique en présence d'un composé basique, pour former les 7-organosulfonyloxy-5-thiaprostaglandines $E_1$ correspondantes, à traiter les 7-organosulfonyloxy-5-thiaprostaglandines $E_1$ résultantes par un composé basique, puis à soumettre éventuellement le produit résultant à une déprotection et/ou à une hydrolyse et/ou à une salification, en formant ainsi des 5-thia-$\Delta^7$-prostaglandines de formule générale (II)

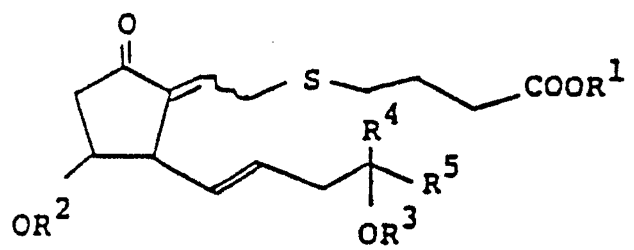

... (II)

dans laquelle $R^1$, $R^2$, $R^3$, $R^4$ et $R^5$ sont tels que définis précédemment et l'indication

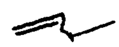

représente une forme Z, une forme E ou un mélange d'une forme Z et d'une forme E dans n'importe quel rapport, à réduire sélectivement le composé (II) résultant et à soumettre éventuellement le produit réduit résultant à une déprotection et/ou à une hydrolyse et/ou à une salification.

**11.** Procédé selon la revendication I0, dans lequel le dérivé réactif de l'acide organique sulfonique est le chlorure de méthanesulfonyle, le chlorure de p-toluènesulfonyle ou l'anhydride d'acide méthanesulfonique.

**12.** Procédé selon la revendication I0, dans lequel le composé basique est une amine.

**Ansprüche**

**1.** 5-Thiaprostaglandine-EI mit der allgemeinen Formel (I):

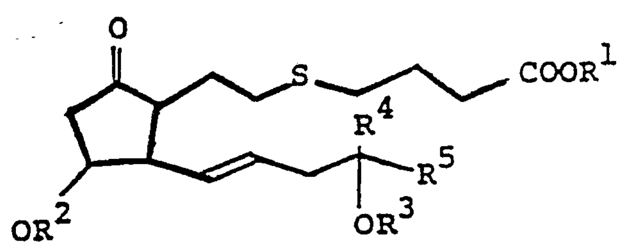

... (I)

wobei $R^1$ für ein Wasserstoffatom, eine $C_1$- bis $C_{10}$-Alkylgruppe, eine substituierte oder unsubstituierte Phenylgruppe, eine substituierte oder unsubstituierte $C_3$- bis $C_{10}$-Cycloalkylgruppe, eine substituierte oder unsubstituierte Phenyl-($C_1$-$C_2$)-Alkylgruppe oder ein äquivalentes Kation steht; wobei $R^2$ und $R^3$, die gleich oder verschieden sein können, für ein Wasserstoffatom, eine Tri-($C_1$-$C_7$)-Kohlenwasserstoff-Silylgruppe oder eine Gruppe stehen, welche zusammen mit einem Sauerstoffatom einer Hydroxylgruppe eine Acetalbindung bildet; wobei $R^4$ für ein Wasserstoffatom, eine Methylgruppe, eine Ethylgruppe oder eine Vinylgruppe steht; und wobei $R^5$ für eine lineare oder verzweigte $C_3$- bis $C_8$-Alkylgruppe, eine lineare oder verzweigte $C_3$- bis $C_8$-Alkenylgruppe, eine lineare oder verzweigte $C_3$-bis $C_8$-Alkynylgruppe, eine Phenylgruppe, die substituiert sein kann, eine Phenoxygruppe, die substituiert sein kann, eine $C_3$- bis $C_{10}$-Cycloalkyl-gruppe, die substituiert sein kann, oder eine lineare oder verzweigte $C_1$- bis $C_5$-Alkylgruppe steht, die mit einer $C_1$- bis $C_6$-Alkoxygruppe, einer Phenylgruppe, die substituiert sein kann, einer Phenoxygruppe, die substituiert sein kann, oder einer $C_3$- bis $C_{10}$-Cycloalkylgruppe, die substituiert sein kann, substituiert ist.

2. 5-Thiaprostaglandine-EI nach Anspruch I, wobei $R^1$ für ein Wasserstoffatom, ein $C_1$- bis $C_{10}$-Alkyl oder ein äquivalentes Kation steht.

3. 5-Thiaprostaglandine-EI nach Anspruch I, wobei $R^2$ und $R^3$ gleich oder verschieden sein können und für ein Wasserstoffatom, eine Tri-($C_1$-$C_4$)-Alkylsilylgruppe, eine Diphenyl-($C_1$-$C_4$)-Alkylsilylgruppe, eine 2-Tetrahydropyranylgruppe, eine 2-Tetrahydrofuranylgruppe, eine I-Ethoxyethylgruppe, eine 2-Ethoxy-2-Propylgruppe, eine (2-Methoxyethoxy)-Methylgruppe oder eine 6,6-Dimethyl-3-Oxa-2-Oxobicyclo-[3,I,0]-Hexa-4-yl-Gruppe stehen.

4. 5-Thiaprostaglandine-EI nach Anspruch I, wobei $R^5$ für eine Butylgruppe, eine Pentylgruppe, eine I-Methyl-I-Butylgruppe, eine 2-Methyl-I-Butylgruppe, eine Cyclopentylgruppe, eine Cyclohexylgruppe oder eine Phenylgruppe steht.

5. 5-Thiaprostaglandine-EI nach Anspruch I, wobei $R^4$ für eine Methylgruppe steht.

6. Verfahren zum Herstellen von 5-Thiaprostaglandinen-EI mit der allgemeinen Formel (I):

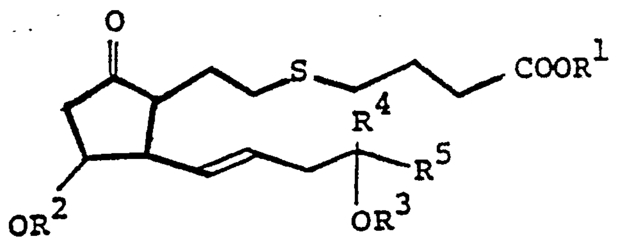

... (I)

wobei $R^1$ für ein Wasserstoffatom, eine $C_1$- bis $C_{10}$-Alkylgruppe, eine substituierte oder unsubstituierte Phenylgruppe, eine substituierte oder unsubstituierte $C_3$- bis $C_{10}$-Cycloalkylgruppe, eine substituierte oder unsubstituierte Phenyl-($C_1$-$C_2$)-Alkylgruppe oder ein äquivalentes Kation steht; wobei $R^2$ und $R^3$, die gleich oder verschieden sein können, für ein Wasserstoffatom, eine Tri-($C_1$-$C_7$)-Kohlenwasserstoff-Silylgruppe oder eine Gruppe stehen, welche zusammen mit einem Sauerstoffatom einer Hydroxylgruppe eine Acetalbindung bildet; wobei $R^4$ für ein Wasserstoffatom, eine Methylgruppe, eine Ethylgruppe oder eine Vinylgruppe steht; und wobei $R^5$ für eine lineare oder verzweigte $C_3$- bis $C_8$-Alkylgruppe, eine lineare oder verzweigte $C_3$- bis $C_8$-Alkenylgruppe, eine lineare oder verzweigte $C_3$-bis $C_8$-Alkynylgruppe, eine Phenylgruppe, die substituiert sein kann, eine Phenoxygruppe, die substituiert sein kann, eine $C_3$- bis $C_{10}$-Cycloalkylgruppe, die substituiert sein kann, oder eine lineare oder verzweigte $C_1$- bis $C_5$-Alkylgruppe steht, die mit einer $C_1$- bis $C_6$-Alkoxygruppe, einer Phenylgruppe, die substituiert sein kann, einer Phenoxygruppe, die substituiert sein kann, oder einer $C_3$- bis $C_{10}$-Cycloalkylgruppe, die substituiert sein kann, substituiert ist,

welches umfaßt:

5-Thia-$\Delta^7$-Prostaglandine-EI mit der allgemeinen Formel (II):

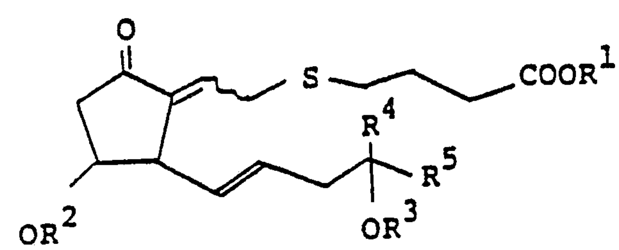

... (II)

wobei für $R^1$, $R^2$, $R^3$, $R^4$ bzw. $R^5$ die oben angegebene Definition gilt und wobei das Zeichen

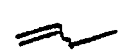

für eine Z-Form, eine E-Form oder eine Mischung von einer Z-Form und einer E-Form in jedem Verhältnis steht,

werden selektiv reduziert und das dabei erhaltende Produkt wird nach Wunsch einer Deprotektion

und/oder Hydrolyse und/oder Salzbildung unterworfen.

7. Verfahren nach Anspruch 6, bei dem die selektive Reduktion in Anwesenheit eines zinkhaltigen Reduziermittels oder einer Organozinn-Hydridverbindung durchgeführt wird.

8. Verfahren nach Anspruch 6, bei dem $R^1$ für eine $C_1$- bis $C_{10}$-Alkylgruppe steht.

9. Verfahren nach Anspruch 6, wobei $R^2$ und $R^3$ gleich oder verschieden sein können und für ein Wasserstoffatom, eine Tri($C_1$-$C_4$)-Alkylsilylgruppe, eine Diphenyl-($C_1$-$C_4$)-Alkylsilylgruppe, eine 2-Tetrahydropyranylgruppe, eine 2-Tetrahydrofuranylgruppe, eine l-Ethoxyethylgruppe, eine 2-Ethoxy-2-Proplgruppe, eine (2-Methoxyethoxy)-Methylgruppe oder eine 6,6-Dimethyl-3-Oxa-2-Oxobicyclo-[3,l,0]-Hexa-4-yl-Gruppe stehen.

10. Verfahren zum Herstellen von 5-Thiaprostaglandinen-El mit der allgemeinen Formel (I):

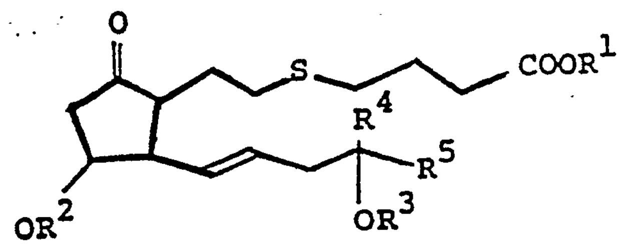

... (I)

wobei $R^1$ für ein Wasserstoffatom, eine $C_1$- bis $C_{10}$-Alkylgruppe, eine substituierte oder unsubstituierte Phenylgruppe, eine substituierte oder unsubstituierte $C_3$- bis $C_{10}$-Cycloalkylgruppe, eine substituierte oder unsubstituierte Phenyl-($C_1$-$C_2$)-Alkylgruppe oder ein äquivalentes Kation steht; wobei $R^2$ und $R^3$, die gleich oder verschieden sein können, für ein Wasserstoffatom, eine Tri-($C_1$-$C_7$)-Kohlenwasserstoff-Silylgruppe oder eine Gruppe stehen, welche zusammen mit einem Sauerstoffatom einer Hydroxylgruppe eine Acetalbindung bildet; wobei $R^4$ für ein Wasserstoffatom, eine Methylgruppe, eine Ethylgruppe oder eine Vinylgruppe steht; und wobei $R^5$ für eine lineare oder verzweigte $C_3$- bis $C_8$-Alkylgruppe, eine lineare oder verzweigte $C_3$- bis $C_8$-Alkenylgruppe, eine lineare oder verzweigte $c_3$-bis $C_8$-Alkynylgruppe, eine Phenylgruppe, die substituiert sein kann, eine Phenoxygruppe, die substituiert sein kann, eine $C_3$- bis $C_{10}$-Cyclo-alkylgruppe, die substituiert sein kann, oder eine lineare oder verzweigte $c_1$- bis $C_5$-Alkylgruppe steht, die mit einer $C_1$- bis $C_6$-Alkoxygruppe, einer Phenylgruppe, die substituiert sein kann, einer Phenoxygruppe, die substituiert sein kann, oder einer $C_3$- bis $C_{10}$-Cycloalkylgruppe, die substituiert sein kann, substituiert ist, welches folgende Schritte umfaßt:

man läßt ein 7-Hydroxy-5-Thiaprostaglandin-El mit folgender allgemeiner Formel (III):

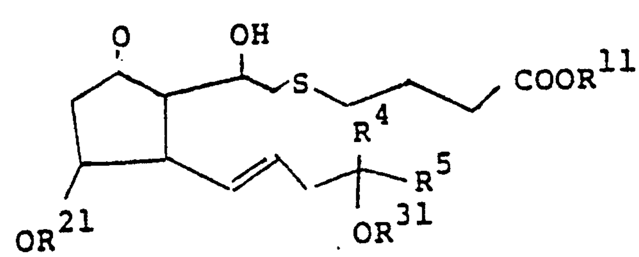

... (III)

wobei für $R^4$ und $R^5$ die oben angegebene Definition gilt, wobei $R^{11}$ für ein Wasserstoffatom, eine $C_1$- bis $C_{10}$-Alkylgruppe, eine substituierte oder unsubstituierte Phenylgruppe, eine substituierte oder unsubstituierte $C_3$- bis $C_{10}$-Cycloalkylgruppe oder eine substituierte oder unsubstituierte Phenyl-($C_1$-$C_2$)-Alkylgruppe steht, und wobei $R^{21}$ und $R^{31}$, die gleich oder verschieden sein können, für eine Tri-($C_1$-$C_7$)-Kohlenwasserstoffsilylgruppe oder eine Gruppe stehen, die mit einem Sauerstoffatom einer Hydroxylgruppe eine Acetalbindung bildet,

mit einem Reaktionsderivat einer organischen Sulfonsäure in Anwesenheit einer basischen Verbindung reagieren, um das entsprechende 7-Organosulfonyloxy-5-Thiaprosta-glandin-El zu bilden;

man behandelt das dabei erhaltene 7-Organosulfo-Nyloxy-5-Thiaprostaglandin-El mit einer basischen Verbindung;

anschließend wird das dabei erhaltende Produkt nach Wunsch einer Deprotektion und/oder Hydrolyse und/oder Salzbildung unterworfen, wobei 5-Thia-$\Delta^7$-Prostaglandine gebildet werden, welche die folgende allgemeine Formel (II) haben:

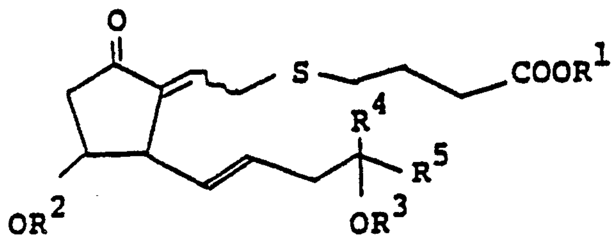

... (II)

wobei für $R^1$, $R^2$, $R^3$, $R^4$ und $R^5$ die oben angegebenen Definitionen gelten und wobei das Zeichen

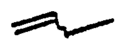

für eine Z-Form, eine E-Form oder eine Mischung einer Z-Form und einer E-Form in jedem beliebigen Verhältnis steht;

die resultierende Verbindung (II) wird selektiv reduziert; und

das resultierende, reduzierte Produkt wird nach Wunsch einer Deprotektion und/oder Hydrolyse und/oder Salzbildung unterworfen.

**11.** Verfahren nach Anspruch l0, bei dem das reaktive Derivat der organischen Sulfonsäure Methansulfonylchlorid, p-Toluolsulfonylchlorid oder Methansulfonsäureanhydrid ist.

**12.** Verfahren nach Anspruch l0, bei dem die basische Verbindung ein Amin ist.